# EUROPEAN PATENT APPLICATION

(11) **EP 4 751 589 A2**
(43) Date of publication of application: **03.06.2026**
(21) Application number: 25208183.1
(22) Date of filing: 13.10.2025
(51) Int. Cl.: A24F 40/40, A24F 40/42, A61M 15/00, A24F 40/10

(54) **SHELL STRUCTURE AND AEROSOL DEVICE**

(30) Priority: 12.10.2024 CN 202422478815 U
(71) Applicant: Shenzhen Relx Technology Co., Ltd., Shenzhen City, Guangdong Province 518108 (CN)
(72) Inventor: GUO, HAOHANG, Shenzhen, 518108 (CN); XU, SHENGYANG, Shenzhen, 518108 (CN)
(74) Representative: Metida

(57) **Abstract**

A shell structure (100a, 100b) includes a mouthpiece (10a, 10b), a connecting component (20a, 20b) on a side of the mouthpiece (10a, 10b), a support component (30a, 30b) configured to support the connecting component (20a, 20b), a first magnetic component (40a, 40b), a second magnetic component (50a, 50b), and a third magnetic component (60a, 60b). The mouthpiece (10a, 10b) includes an inhalation channel (11a). The connecting component (20a, 20b) includes an airflow channel (21a, 21b) connected to an air inlet end (111a) of the inhalation channel (11a). The first, second, third magnetic components (40a, 40b, 50a, 50b, 60a, 60b) are arranged on the connecting component (20a, 20b), the mouthpiece (10a, 10b), and the support component (30a, 30b), respectively. The mouthpiece (10a, 10b) is magnetically connected to the connecting component (20a, 20b) through a magnetic force between the third and first magnetic components (60a, 60b, 40a, 40b).

## Description

### FIELD

The present invention relates to field of aerosol device, and in particular to a shell structure and an aerosol device.

### BACKGROUND

An Aerosol device generally consists of a mouthpiece, a support rod, and a main shell, etc. The mouthpiece, the support rod, and the main shell are mostly connected by means of snap-fit or screw connection, which causes the disassembly and assembly of the various components of the aerosol device cumbersome and is not conducive to the replacement of the aerosol generation assembly of the aerosol device.

### SUMMARY

To solve the problem of cumbersome disassembly and assembly of the mouthpiece, the support rod, and the main shell in the aerosol device, it is necessary to provide a shell structure and an aerosol device having the shell structure.

The present application provides a shell structure applied in an aerosol device. The shell structure includes a mouthpiece, a connecting component, a support component, a first magnetic component, a second magnetic component, and a third magnetic component. The mouthpiece is provided with an inhalation channel. The connecting component is arranged on a side of the mouthpiece, the connecting component is provided with an airflow channel, and the airflow channel is connected to an air inlet end of the inhalation channel. The support component is configured to support the connecting component. The first magnetic component is arranged on the connecting component. The second magnetic component is arranged on the mouthpiece. The third magnetic component is arranged on the support component. At least one of the connecting component and the support component is configured to accommodate an aerosol generation assembly of the aerosol device, the airflow channel is configured to receive an aerosol generated by the aerosol generation assembly, the mouthpiece is magnetically connected to the connecting component through a magnetic force between the third magnetic component and the first magnetic component.

In some possible implementations, the shell structure includes at least two first magnetic components including the first magnetic component, at least two second magnetic components including the second magnetic component, and at least two third magnetic components including the third magnetic component. The at least two second magnetic components correspond one-to-one to the at least two first magnetic components, and the at least two third magnetic components correspond one-to-one to the at least two first magnetic components.

In some possible implementations, a first accommodation slot is arranged on an end surface of the connecting component close to the mouthpiece, the first magnetic component is located in the first accommodation slot. An installation part protrudes from an end of the mouthpiece close to the connecting component and extends into the first accommodation slot or is located outside the connecting component, and the first magnetic component is arranged an end surface of the installation part close to the connecting component.

In some possible implementations, the connecting component is provided with at least two airflow channels, the mouthpiece is configured to be rotatable relative to the connecting component, thereby allowing the inhalation channel of the mouthpiece to be selectively connected to any one of the at least two airflow channels.

In some possible implementations, the shell structure defines a rotation axis, the mouthpiece is rotatable around the rotation axis, the number of the at least two airflow channels is 2N, N is a positive integer, the shell structure includes N groups of first magnetic components including the first magnetic component, each group of the N groups of first magnetic components includes at least two first magnetic components, the at least two first magnetic components includes two first magnetic components symmetrically arranged with respect to the rotation axis.

In some possible implementations, the shell structure defines a rotation axis, the mouthpiece is rotatable around the rotation axis, the number of the at least two airflow channels is 2N+1, N is a positive integer, the shell structure includes N+1 groups of first magnetic components including the first magnetic component, each group of the N+1 groups of first magnetic components includes at least one first magnetic component, the N+1 groups of first magnetic components are evenly spaced around the rotation axis.

In some possible implementations, the shell structure further includes a rotating component and a rotating base, the rotating component is connected to a side of the mouthpiece close to the connecting component, the rotating base is connected to the connecting component. The rotating base includes a rotating groove, and at least part of the rotating component is rotatably arranged in the rotating groove.

In some possible implementations, the shell structure further includes a limiting structure, the rotating component rotatably cooperates with the rotating groove, the limiting structure is configured to restrict a movement of the rotating component along an axis of the rotating component.

In some possible implementations, the limiting structure further includes a first limiting part and a second limiting part, the first limiting part is arranged on the rotating component, the second limiting part is arranged on the rotating base, the first limiting part and the second limiting part abut against each other along the axis of the rotating component when the rotating component rotatably cooperates with the rotating groove.

In some possible implementations, an inner circumferential surface of the rotating groove defines a lubrication groove, and the lubrication groove is configured to store lubricating medium.

In some possible implementations, a blocking part protrudes from the side of the mouthpiece close to the connecting component, the blocking part is arranged around an outer circumferential surface of the rotating component, the blocking part is spaced apart from the outer circumferential surface of the rotating component to form an activity cavity between the blocking part and the rotating component, the rotating base is movably arranged in the activity cavity. The activity cavity is configured to store the lubricating medium overflowing from the rotating groove.

In some possible implementations, the shell structure further includes an oil-absorbing member, the oil-absorbing member is connected to the mouthpiece or the connecting component, the oil-absorbing member is configured to absorb the lubricating medium overflowing from the rotating groove, and/or the oil-absorbing member is configured to absorb condensate liquid formed after the aerosol condenses.

In some possible implementations, the shell structure further includes an oil-absorbing member, the oil-absorbing member is connected to the side of the mouthpiece close to the connecting component, the oil-absorbing member is configured to absorb condensate liquid formed after the aerosol condenses, a through hole is formed in the oil-absorbing member and connects the airflow channel and the inhalation channel.

In some possible implementations, the mouthpiece is configured to be rotatable relative to the connecting component, the oil-absorbing member is rotatable relative to the connecting component during a rotation of the mouthpiece. The oil-absorbing member is configured to absorb the condensate liquid on an end surface of the connecting component close to the mouthpiece during a rotation of the oil-absorbing member relative to the connecting component.

The present application also provides an aerosol device including the above shell structure and an aerosol generation assembly. The aerosol generation assembly are removably accommodated in the connection component of the shell structure or the support component of the shell structure.

In the present application, the shell structure is composed of the mouthpiece, the connecting component and the support component. The first magnetic component is arranged on the connecting component, the second magnetic component is arranged on the mouthpiece, and the third magnetic component is arranged on the support component. The mouthpiece is magnetically connected to the connecting component through the magnetic force between the second magnetic component and the first magnetic component, and the support component is magnetically connected to the connecting component through the magnetic force between the third magnetic component and the first magnetic component. Thus, the mouthpiece, the connecting component and the support component are magnetically connected through three groups of magnetic components, which is convenient to quickly disassemble and assemble any one of the mouthpiece, the connecting component and the support component, and is convenient for users to operate. In addition, in the shell structure of the present application, only one group of the first magnetic component is arranged on the connecting component, and the second magnetic component on the mouthpiece 10a and the third magnetic component on the support component are simultaneously magnetically connected to this group of the first magnetic component. Compared with the existing technology in which two groups of the first magnetic component are arranged on the connecting component to be magnetically connected to the second magnetic component and the third magnetic component respectively, not only the cost of the shell structure is reduced, but also the assembly process of the shell structure is simplified.

### BRIEF DESCRIPTION OF THE DRAWINGS

FIG. 1 is a schematic structure diagram of a shell structure of Embodiment 1 of the present application.
FIG. 2 is an exploded view of the shell structure of FIG. 1.
FIG. 3 is a cross-sectional view of the shell structure taken along I-I in FIG. 1.
FIG. 4 is a cross-sectional view of the shell structure taken along II-II in FIG. 1.
FIG. 5 is a top view of a connecting component of the shell structure of another embodiment of the present application.
FIG. 6 is a top view of the connecting component of the shell structure of another embodiment of the present application.
FIG. 7 is a top view of the connecting component of the shell structure of another yet embodiment of the present application.
FIG. 8 is a schematic structure diagram of a mouthpiece of the shell structure of FIG. 1.
FIG. 9 is a cross-sectional view of the shell structure taken along III-III in FIG. 1.
FIG. 10 is a structural schematic diagram of the connecting component of the shell structure of FIG. 1.
FIG. 11 is a bottom view of the mouthpiece of the shell structure of another embodiment of the present application.
FIG. 12 is a top view of the connecting component of the shell structure of another embodiment of the present application.
FIG. 13 is a structural schematic diagram of the connecting component of the shell structure of the Embodiment 1 of the present application in another perspective.
FIG. 14 is a structural schematic diagram of an aerosol device of the Embodiment 1 of the present application.
FIG. 15 is an exploded view of the aerosol device of the first embodiment of the present application.
FIG. 16 is a structural schematic diagram of the shell structure of Embodiment 2 of the present application.
FIG. 17 is an exploded view of the shell structure of the Embodiment 2of the present application.
FIG. 18 is a cross-sectional view of the shell structure taken along IV-IV in FIG. 16.
FIG, 19 is an exploded view of the aerosol device of the Embodiment 2 of the present application.

### DETAILED DESCRIPTION

The following description will refer to the attached figures to provide a more comprehensive description of the content of this application. The figures show exemplary embodiments of this application. However, this application can be implemented in many different forms and should not be construed as being limited to the exemplary embodiments described herein. These exemplary embodiments are provided to make this application thorough and complete and to convey the scope of this application to those skilled in the art. Similar reference numerals denote the same or similar components.

The terms used herein are only for the purpose of describing specific exemplary embodiments and are not intended to limit this application. As used herein, unless the context clearly indicates otherwise, the singular forms "a", "an" and "the" are intended to include the plural forms as well. Furthermore, when used herein, "comprising" and/or "including" and/or "having", integers, steps, operations, components and/or components, but do not exclude the existence or addition of one or more other features, regions, integers, steps, operations, components and/or groups thereof.

Unless otherwise defined, all terms used herein (including technical and scientific terms) have the same meaning as commonly understood by those skilled in the art to which this application belongs. In addition, unless otherwise defined herein, such terms as those defined in general dictionaries should be interpreted as having meanings consistent with their meanings in the relevant technology and the context of this application, and will not be interpreted as idealized or overly formal meanings.

The following description of the specific embodiments of this application will be made with reference to the attached figures.

### Embodiment 1

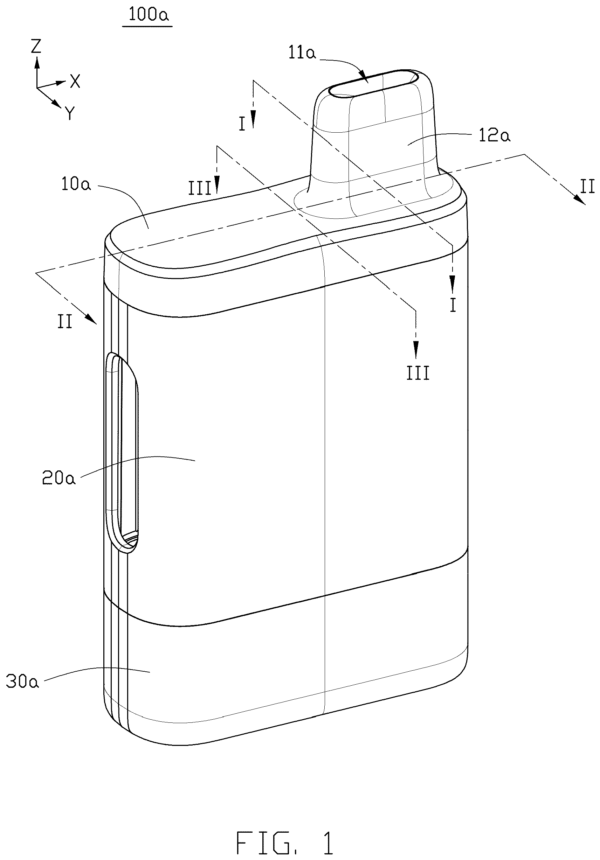

Referring to FIGS. 1 to 4 and FIGS. 14 and 15, the Embodiment 1 provides a shell structure 100a, applied to an aerosol device 200a. The aerosol device 200a includes an aerosol generation assembly 6a and a shell structure 100a, the aerosol generation assembly 6a is installed within the shell structure 100a. The aerosol generation assembly 6a of the aerosol device 200a can atomize an atomizable substrate into aerosol for the user to inhale.

The aerosol device 200a may be an electronic cigarette, correspondingly, the atomizable substrate may be e-liquid.

It can be understood that the aerosol device 200a may be an aroma diffuser, correspondingly, the atomizable substrate may be aroma essential oil, etc. The aerosol device 200a may be a medical nebulizer, correspondingly, the atomizable substrate may be therapeutic liquid medicine, etc. The aerosol device 200a may be a disinfection device, correspondingly, the atomizable substrate may be solid disinfection powder, etc.

For the convenience of subsequent reading, the present application introduces a first direction Z, a second direction X and a third direction Y to describe the embodiments of the present application. The first direction Z, the second direction X and the third direction Y can be three non-parallel straight line directions in space; further, the first direction Z, the second direction X and the third direction Y can be three mutually perpendicular directions in a three-dimensional coordinate system (three-dimensional Cartesian coordinate system). In the subsequent embodiments, the description will be made by taking the first direction Z as the Z-axis direction of the coordinate axes of the three-dimensional coordinate system, the second direction X as the X-axis direction of the coordinate axes of the three-dimensional coordinate system, and the third direction Y as the Y-axis direction of the coordinate axes of the three-dimensional coordinate system as an example.

The shell structure 100a includes a mouthpiece 10a, a connecting component 20a, a support component 30a, a first magnetic component 40a, a second magnetic component 50a, and a third magnetic component 60a. Along the first direction Z, the mouthpiece 10a, the connecting component 20a and the support component 30a are arranged in sequence. The mouthpiece component 10a is provided with an inhalation channel 11a, and the inhalation channel 11a includes an air inlet end 111a and an air outlet end 112a arranged relatively along the first direction Z. The connecting component 20a is located on a side of the mouthpiece 10a, and the connecting component 20a is provided with an airflow channel 21a, the airflow channel 21a is connected to the air inlet end 111a of the inhalation channel 11a and communicates with the air inlet end 111a. The support component 30a is configured to support the connecting component 20a.

The first magnetic component 40a is arranged on the connecting component 20a, the second magnetic component 50a is arranged on the mouthpiece 10a, and the third magnetic component 60a is arranged on the support component 30a. At least one of the connecting component 20a and the support component 30a is configured to accommodate the aerosol generation assembly 6a, and the airflow channel 21a is configured to receive part of the aerosol generated by the aerosol generation assembly 6a. The part of the aerosol enters the air inlet end 111a of the inhalation channel 11a from the airflow channel 21a, and then leaves the inhalation channel 11a from the air outlet end 112a of the inhalation channel 11a and is inhaled by the user.

The mouthpiece 10a is magnetically connected to the connecting component 20a through a magnetic force between the second magnetic component 50a and the first magnetic component 40a, and the support component 30a is magnetically connected to the connecting component 20a through a magnetic force between the third magnetic component 60a and the first magnetic component 40a.

Thus, in the present application, the shell structure 100a is composed of the mouthpiece 10a, the connecting component 20a and the support component 30a. The first magnetic component 40a is arranged on the connecting component 20a, the second magnetic component 50a is arranged on the mouthpiece 10a, and the third magnetic component 60a is arranged on the support component 30a. The mouthpiece 10a is magnetically connected to the connecting component 20a through the magnetic force between the second magnetic component 50a and the first magnetic component 40a, and the support component 30a is magnetically connected to the connecting component 20a through the magnetic force between the third magnetic component 60a and the first magnetic component 40a. Thus, the mouthpiece 10a, the connecting component 20a and the support component 30a are magnetically connected through three groups of magnetic components, which is convenient to quickly disassemble and assemble any one of the mouthpiece 10a, the connecting component 20a and the support component 30a, and is convenient for users to operate. In addition, in the shell structure 100a of the present application, only one group of the first magnetic component 40a is arranged on the connecting component 20a, and the second magnetic component 50a on the mouthpiece 10a and the third magnetic component 60a on the support component 30a are simultaneously magnetically connected to this group of the first magnetic component 40a. Compared with the existing technology in which two groups of the first magnetic component 40a are arranged on the connecting component 20a to be magnetically connected to the second magnetic component 50a and the third magnetic component 60a respectively, not only the cost of the shell structure 100a is reduced, but also the assembly process of the shell structure 100a is simplified.

Referring to FIGS. 1 to 4 and FIGS. 14 and 15, in some embodiments, the connecting component 20a is provided with at least two airflow channels 21a, and the mouthpiece 10a is configured to be rotatable relative to the connecting component 20a, so that the inhalation channel 11a of the mouthpiece 10a can be selectively connected to any one of the at least two airflow channels 21a.

Each of the airflow channels 21a receives aerosol generated by one aerosol generation assembly 6a, and the inhalation channel 11a is connected to different airflow channels 21a through the rotation of the mouthpiece 10a, thereby allowing aerosol generated by different aerosol generation assemblies 6a to enter the inhalation channel 11a and be inhaled.

It can be understood that the aerosol generation assemblies 6a in the at least two airflow channels 21a may be the same aerosol generation assemblies, so that when the current aerosol generation assembly 6a is used up, another aerosol generation assembly 6a can be directly switched to continue use.

In some embodiments, different aerosol generation assemblies 6a may be arranged in the at least two airflow channels 21a, so that the user can switch different aerosol generation assemblies 6a at any time.

In some embodiments, the number of the first magnetic components 40a is set to at least two, the number of the second magnetic components 50a is set to at least two, and the number of the third magnetic components 60a is set to at least two. The at least two second magnetic components 50a correspond one-to-one to the at least two first magnetic components 40a, and the at least two third magnetic components 60a correspond one-to-one to the at least two first magnetic components 40a. Thus, the arrangement of the at least two first magnetic components 40a, the at least two second magnetic components 50a, and the at least two third magnetic components 60a may increase the number of magnetic components to improve the stability of the connection between the mouthpiece 10a, the connecting component 20a, and the support component 30a.

In addition, in the illustrated embodiment, the number of the first magnetic components 40a, the number of the second magnetic components 50a, and the number of the third magnetic components 60a are the same as the number of the airflow channels 21a, the first magnetic components 40a correspond one-to-one to the airflow channels 21a, the second magnetic components 50a correspond one-to-one to the airflow channels 21a, and the third magnetic components 60a correspond one-to-one to the airflow channels 21a. Thus, when the mouthpiece 10a rotates to different positions relative to the connecting component 20a to connect the inhalation channel 11a to different airflow channels 21a, the second magnetic components 50a also rotate to a preset position along with the mouthpiece 10a and are magnetically connected to the corresponding first magnetic components 40a, so that the mouthpiece 10a at different positions may be stably connected to the connecting component 20a.

Referring to FIGS. 1 to 4, in some embodiments, the number of the airflow channels 21a is set to two, and the two airflow channels 21a are spaced apart along the second direction X. Correspondingly, the number of the first magnetic components 40a, the number of the second magnetic components 50a, and the number of the third magnetic components 60a are also set to two.

The shell structure 100a has a defined rotation axis H, and the mouthpiece 10a can rotate relative to the connecting component 20a around the rotation axis H. The two second magnetic components 50a are respectively arranged on opposite sides of the rotation axis H, and the two second magnetic components 50a are symmetrically arranged with the rotation axis H as the reference. That is, a connection line between center points of the two second magnetic components 50a intersects with the rotation axis H, so that an arc angle of the two second magnetic components 50a relative to the rotation axis H is the same as an arc angle of the two airflow channels 21a relative to the rotation axis H and is 180°. In addition, the connection line between the center points of the two second magnetic components 50a may be parallel to the second direction X, or the connection line between the center points of the two second magnetic components 50a may intersect with the second direction X.

Correspondingly, positions of the two first magnetic components 40a correspond to positions of the two second magnetic components 50a, respectively.

Thus, when the inhalation channel 11a of the mouthpiece 10a is connected to one of the air flow channels 21a, the two second magnetic components 50a are respectively magnetically connected to the two first magnetic components 40a. When the mouthpiece 10a rotates 180° around the rotation axis H relative to the connecting component 20a and the inhalation channel 11a is connected to the other air flow channel 21a, the two second magnetic components 50a rotate 180° synchronously with the mouthpiece 10a. One of the two second magnetic components 50a rotates to be magnetically connected to the other first magnetic component 40a, and the other second magnetic component 50a rotates to be magnetically connected to the remaining first magnetic component 40a, thereby ensuring that when the mouthpiece 10a rotates to connect the inhalation channel 11a with any one of the air flow channels 21a, one of the two second magnetic components 50a is magnetically connected to one of the two first magnetic components 40a, and the other second magnetic component 50a is magnetically connected to the other first magnetic component 40a. This not only realizes the connection between the mouthpiece 10a and the connecting component 20a, but also ensures that the mouthpiece 10a can rotate to the correct position under the assistance of the magnetic force between the first magnetic components 40a and the second magnetic components 50a, so that the inhalation channel 11a and the air flow channel 21a can be quickly and accurately connected.

Furthermore, the two third magnetic components 60a are correspondingly arranged with the two first magnetic components 40a, so that one of the two third magnetic components 60a, one of the two first magnetic components 40a and one of the two second magnetic components 50a are arranged in series in the first direction Z, and the other third magnetic component 60a, the other first magnetic component 40a and the other second magnetic component 50a are also arranged in series in the first direction Z, which can ensure that a relatively stable magnetic connection relationship is formed between the first magnetic component 40a, the second magnetic component 50a and the third magnetic component 60a, thereby improving the stability of the connection between the mouthpiece 10a, the connecting component 20a and the support component 30a. The first magnetic components 40a, the second magnetic components 50a and the third magnetic components 60a are all made of magnetic materials.

In the illustrated embodiment, the first magnetic component 40a is approximately in a cylindrical shape, and its cross-sectional shape is circular. It can be understood that the cross-sectional shape of the first magnetic component 40a may be rectangular, triangular or other shapes. Outer shapes of the second magnetic component 50a and the third magnetic component 60a are approximately the same as that of the first magnetic component 40a.

Along the first direction Z, magnetic poles at opposite ends of the first magnetic component 40a are respectively N pole and S pole, magnetic poles at opposite ends of the second magnetic component 50a are respectively N pole and S pole, and magnetic poles at opposite ends of the third magnetic component 60a are respectively N pole and S pole. The N pole of the first magnetic component 40a is in contact with the S pole of the second magnetic component 50a, and the S pole of the first magnetic component 40a is in contact with the N pole of the third magnetic component 60a.

In some embodiments, along the first direction Z, the second magnetic component 50a and the third magnetic component 60a may be spaced from the first magnetic component 40a, respectively, so that the user can more easily overcome the magnetic force to rotate the mouthpiece 10a. However, a spacing between the second magnetic component 50a and the first magnetic component 40a and a spacing between the third magnetic component 60a and the first magnetic component 40a do not exceed 0.3mm, respectively, which ensure that a magnetic force between the second magnetic component 50a and the first magnetic component 40a and a magnetic force between the third magnetic component 60a and the first magnetic component 40a may maintain the stability of the connection between the mouthpiece 10a, the connecting component 20a and the support component 30a.

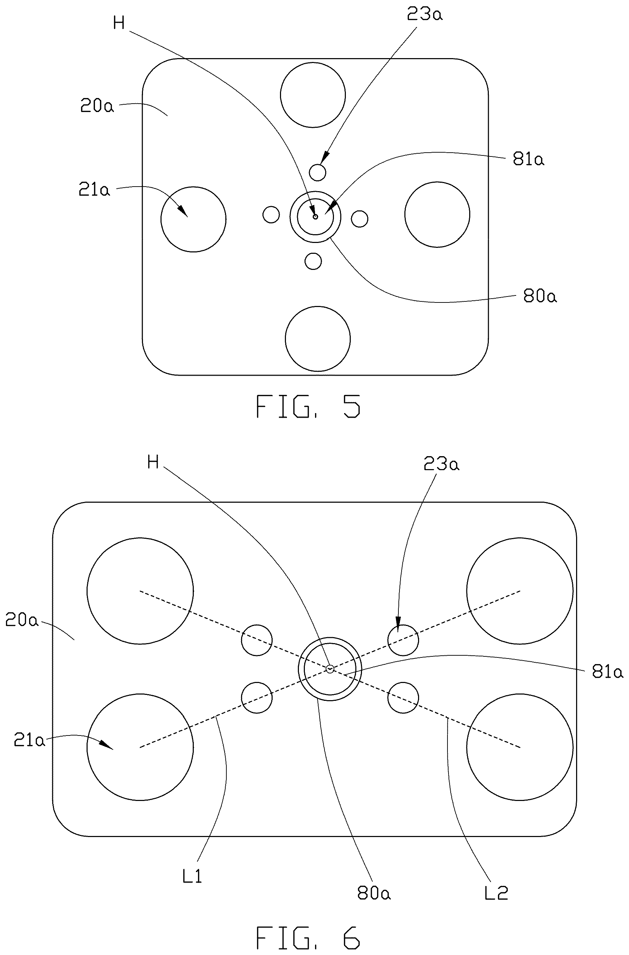

Referring to FIGS. 5 to 7, and FIGS. 2 and 3, in some embodiments, the number of air flow channels 21a is set to 2N. Wherein, N is a positive integer, that is, N is 1, 2, 3, etc. The first magnetic components 40a is set as N groups, and each group of the first magnetic components 40a in the N groups of the first magnetic components 40a includes at least two first magnetic components 40a, and the at least two first magnetic components 40a includes two first magnetic components 40a symmetrically arranged with respect to the rotation axis H.

Correspondingly, the number of the second magnetic components 50a is the same as that of the first magnetic components 40a, and the second magnetic components 50a are also set as N groups. The number and position of the second magnetic components 50a in each group of the second magnetic components 50a in the N groups of the second magnetic components 50a are the same as those of a corresponding group of the first magnetic components 40a. When the mouthpiece 10a rotates to connect the inhalation channel 11a with any one of the 2N airflow channels 21a, any group of the second magnetic components 50a in the N groups of second magnetic components 50a can rotate with the mouthpiece 10a to be magnetically connected with a group of the first magnetic components 40a, thereby ensuring that the mouthpiece 10a can rotate to connect the inhalation channel 11a with any one of the 2N airflow channels 21a and guaranteeing the stability of a connection between the inhalation channel 11a and the airflow channel 21a.

Correspondingly, the number of the third magnetic components 60a is the same as that of the first magnetic components 40a, and positions of the third magnetic components 60a correspond one-to-one to positions of the first magnetic components 40a, so as to improve the stability of the connection between the connecting component 20a and the support component 30a through the magnetic connection between multiple third magnetic components 60a and multiple first magnetic components 40a.

It can be understood that in other embodiments, the number of the third magnetic components 60a may be less than the number of the first magnetic components 40a, as long as each of the third magnetic components 60a can be magnetically connected with a corresponding first magnetic component 40a.

The number of the airflow channels 21a is set to four (shown in FIG. 5), that is, the N value is 2. The first magnetic components 40a are two groups, and each group of the first magnetic components 40a has two first magnetic components 40a. The two first magnetic components 40a in any group of the first magnetic components 40a are symmetrically arranged with respect to the rotation axis H. Correspondingly, the second magnetic components 50a are two groups, and each group of the second magnetic components 50a has two second magnetic components 50a.

Correspondingly, the number of the third magnetic components 60a is the same as that of the first magnetic components 40a, and the positions of the third magnetic components 60a correspond one-to-one to positions of the first magnetic components 40a, so as to improve the stability of the connection between the connecting component 20a and the support component 30a through the magnetic connection between multiple third magnetic components 60a and multiple first magnetic components 40a.

It can be understood that in other embodiments, the number of the third magnetic components 60a may be less than the number of the first magnetic components 40a, as long as each of the third magnetic components 60a can be magnetically connected with a corresponding first magnetic component 40a.

The four airflow channels 21a are arranged at equal intervals around the rotation axis H, and the four second magnetic components 50a and the four first magnetic components 40a are arranged at equal intervals around the rotation axis H, which ensures that when the mouthpiece 10a rotates 90 degrees and the inhalation channel 11a is connected to one of the airflow channels 21a, the four second magnetic components 50a can rotate to once again magnetically connect with the four first magnetic components 40a.

It can be understood that in other embodiments, each group of the first magnetic components 40a can also be set to have four or six or other even number of first magnetic components 40a.

Each group of the first magnetic components 40a has four first magnetic components 40a, and a connection line of center points of the four first magnetic components 40a is a straight line. Two first magnetic components 40a in a same group of the first magnetic components 40a are symmetrically arranged with respect to the rotation axis H with the other two first magnetic components 40a in the same group of the first magnetic components 40a.

It can be understood that in other embodiments, the four airflow channels 21a may not be set at equal intervals (shown in FIG. 6).

Two of the four airflow channels 21a are symmetrically arranged with respect to the rotation axis H, and a connection line of center points of the two airflow channels 21a is defined as a first line L1. The other two of the four airflow channels 21a are also symmetrically arranged with respect to the rotation axis H, and a connection line of center points of the other two of the four airflow channels 21a is defined as a second line L2. The first line L1 and the second line L2 are set at an angle, and the angle between them is greater than 0° and less than 90°.

Correspondingly, two first magnetic components 40a of the four first magnetic components 40a are symmetrically arranged with respect to the rotation axis H, and a connection line of center points of the two first magnetic components 40a coincides with the first line L1. The other two first magnetic components 40a of the four first magnetic components 40a are also symmetrically arranged with respect to the rotation axis H, and t a connection line of center points of the other two first magnetic components 40a coincides with the second line L2.

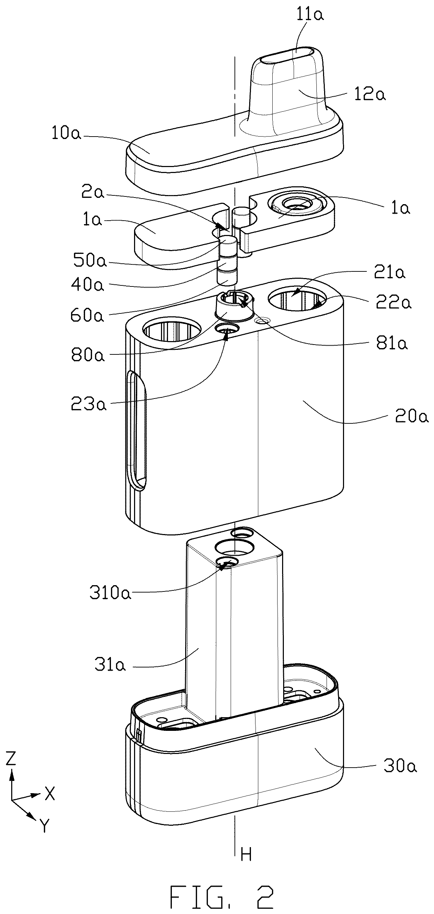

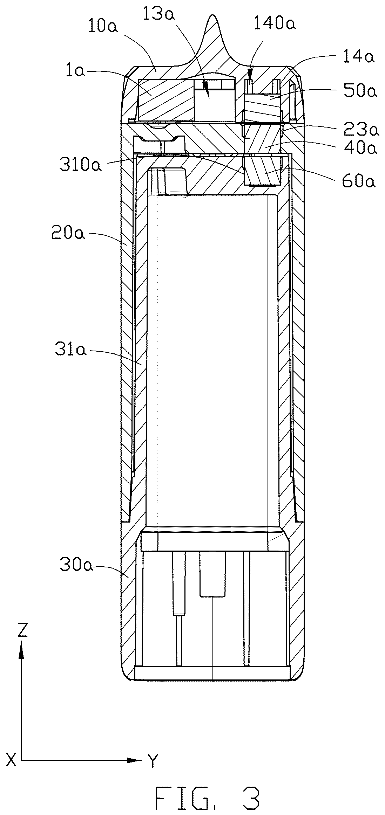

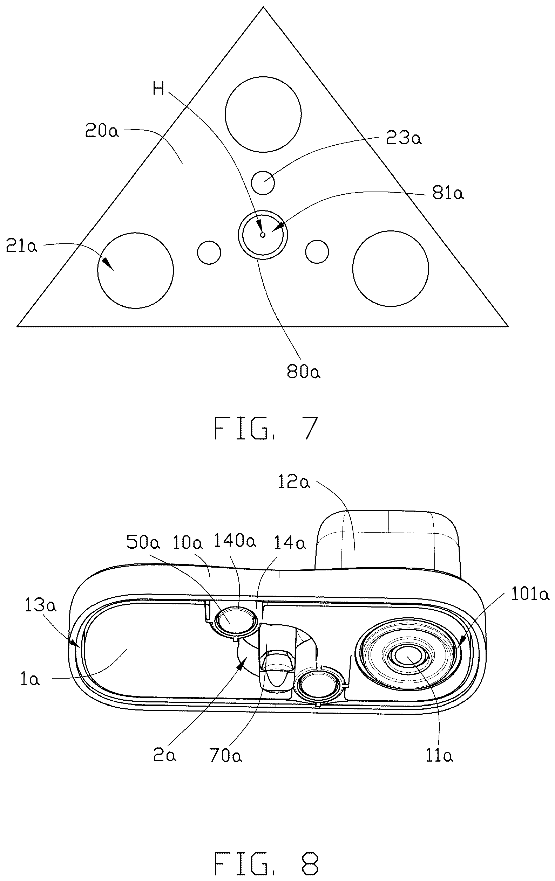

In conjunction with FIG. 7 again, and referring to FIGS. 2 and 3, in some embodiments, the number of airflow channels 21a is set to 2N+1, wherein N is a positive integer, such as 1, 2, 3, etc. The first magnetic components 40a are set to 2N+1 groups, and each group of the first magnetic components 40a includes at least one first magnetic component 40a. The 2N+1 groups of the first magnetic components 40a are evenly spaced around the rotation axis H.

Correspondingly, the number of second magnetic components 50a is the same as that of the first magnetic components 40a, and the second magnetic components 50a are also set to 2N+1 groups. The number and positions of the second magnetic components 50a in each group second magnetic components 50a of the 2N+1 groups of the second magnetic components 50a are the same as those of a corresponding group of first magnetic components 40a. When the mouthpiece 10a rotates to connect the inhalation channel 11a with any one of the 2N+1 airflow channels 21a, any group of the second magnetic components 50a of the 2N+1 groups of the second magnetic components 50a can rotate with the mouthpiece 10a to be magnetically connected with a group of the first magnetic components 40a, thereby ensuring that the mouthpiece 10a can rotate to connect the inhalation channel 11a with any one of the 2N+1 airflow channels 21a and guaranteeing the stability of the connection between the inhalation channel 11a and the airflow channels 21a.

Correspondingly, the number of third magnetic components 60a is the same as that of the first magnetic components 40a, and the positions of the third magnetic components 60a correspond one-to-one to the positions of the first magnetic components 40a, so as to improve the stability of the connection between the connecting component 20a and the support component 30a through the magnetic connection between multiple third magnetic components 60a and multiple first magnetic components 40a.

It can be understood that in other embodiments, the number of third magnetic components 60a may be less than that of the first magnetic components 40a, as long as each third magnetic component 60a can be magnetically connected to a corresponding first magnetic component 40a.

As shown in FIG. 7, the number of airflow channels 21a is set to three, that is, the N value is 1. The three airflow channels 21a are evenly spaced around the rotation axis H. The first magnetic components 40a are three groups, and each group of the first magnetic components 40a includes one first magnetic component 40a. The total number of first magnetic components 40a is three, and the three first magnetic components 40a are evenly spaced around the rotation axis H.

The three first magnetic components 40a are correspondingly arranged with the three airflow channels 21a, that is, a connection line of a center point of any airflow channel 21a and a center point of a corresponding first magnetic component 40a intersects with the rotation axis H.

It can be understood that in other embodiments, the three first magnetic components 40a and the three air flow channels 21a may be arranged alternately around the rotation axis H, that is, there is one air flow channel 21a at a middle position between any two adjacent first magnetic components 40a of the three first magnetic components 40a.

It can be understood that in other embodiments, each group of the first magnetic components 40a may include two or more first magnetic components 40a, and the connection line of the center points of each first magnetic component 40a in each group of the first magnetic components 40a intersects with the rotation axis H.

In conjunction with FIGS. 8 to 10 again and referring to FIG. 1, in some embodiments, the shell structure 100a may further include a rotating component 70a and a rotating base 80a. The rotating component 70a is connected to a side of the mouthpiece 10a close to the connecting component 20a, and the rotating base 80a is connected to the connecting component 20a. The rotating base 80a includes a rotating groove 81a, and at least part of the rotating component 70a is rotatably arranged in the rotating groove 81a.

In some embodiments, the mouthpiece 10a is approximately in a columnar shape, and an end surface of the mouthpiece 10a close to the connecting component 20a is provided with an empty slot 13a. Along the first direction Z, the rotating component 70a protrudes from a bottom wall of the empty slot 13a towards the connecting component 20a. The rotating component 70a is in a cylindrical shape, and an axis of the rotating component 70a coincides with the rotating axis H. The rotating component 70a is integrally formed with the mouthpiece 10a or is detachably connected to the mouthpiece 10a.

Along the first direction Z, a protruding length of the rotating component 70a is greater than a depth of the empty slot 13a, so that an end of the rotating component 70a close to the connecting component 20a can extend out of the empty slot 13a and cooperate with the rotating base 80a.

The rotating base 80a is connected to an end surface of the connecting component 20a close to the mouthpiece 10a, and the rotating base 80a is integrally formed with the connecting component 20a or is detachably connected to the connecting component 20a. The rotating base 80a may be cylindrical or prismatic, etc. Along the first direction Z, the rotating groove 81a extends from an end surface of the rotating base 80a close to the mouthpiece 10a towards a side of the rotating base 80a away from the mouthpiece 10a.

In the illustrated embodiment, a suction part 12a protrudes from an end surface of the mouthpiece 10a facing away from the connecting component 20a. Along the first direction Z, the inhalation channel 11a extends from an end surface of the suction part 12a away from the mouthpiece 10a towards the mouthpiece 10a and penetrates the suction part 12a and the mouthpiece 10a.

In conjunction with FIGS. 8 to 10 again and referring to FIG. 1, in some embodiments, the shell structure 100a may further include a limiting structure 90a. When the rotating component 70a rotatably cooperates with the rotating groove 81a, the limiting structure 90a is configured to restrict a movement of the rotating component 70a along the axis of the rotating component 70a.

The limiting structure 90a includes a first limiting part 91a and a second limiting part 92a. The first limiting part 91a is arranged on the rotating component 70a, and the second limiting part 92a is arranged on the rotating base 80a. When the rotating component 70a rotatably cooperates with the rotating groove 81a, the first limiting part 91a and the second limiting part 92a abut against each other along the axis of the rotating component 70a to prevent the rotating component 70a from moving towards a side of the rotating base 80a close to the mouthpiece 10a along the first direction Z and disengaging from the rotating groove 81a.

In the illustrated embodiment, the rotating component 70a may include a first portion71a and two second portions 72a. An end of the first portion71a is connected to the bottom wall of the empty slot 13a, and the two second portions 72a are connected to another end of the first portion 71a. Along the third direction Y, the two second portions 72a are spaced apart from each other, so that the two second portions 72a may be brought closer to each other under an action of an external force.

The number of the first limiting parts 91a is set to two, and opposite sides of the two second portions 72a are respectively connected to one of the two first limiting parts 91. That is, a side of one of the two second portions 72a facing away from the other of the two second portions 72a is connected to one of the two first limiting parts 91, and a side of the other of the two second portions 72a facing away from the two second portions 72a is connected to the other of the two first limiting parts 91.

A cross-sectional shape of the rotating groove 81a may be stepped, and the rotating groove 81a includes a first groove body 811a and a second groove body 812a that are connected to each other. The second groove body 812a is connected to an end of the first groove body 811a away from the mouthpiece 10a, and a diameter of the first groove body 811a is less than that of the second groove body 812a, thereby forming a step surface at the rotating base 80a, which is the second limiting part 92a.

Each of the two first limiting parts 91a is in a hemispherical shape. Along the third direction Y, a distance between the opposite sides of the two first limiting parts 91a is greater than a diameter of the first groove body 811a and less than a diameter of the second groove body 812a. In this way, when the rotating component 70a is installed on the rotating base 80a, the two second portions 72a are elastically deformed by an abutment force of a wall of the first groove body 811a and move closer to each other, thereby allowing the two second portions 72a to smoothly pass through the first groove body 811a and enter the second groove body 812a. Subsequently, the two second portions 72a are no longer subjected to the abutment force of the wall of the first groove 811a and return to their original position, thereby allowing ends of the two second components 72a closer to the mouthpiece 10a in the first direction Z to abut against the step surface, thereby preventing the second portions 72a from detaching from the second groove body 812a.

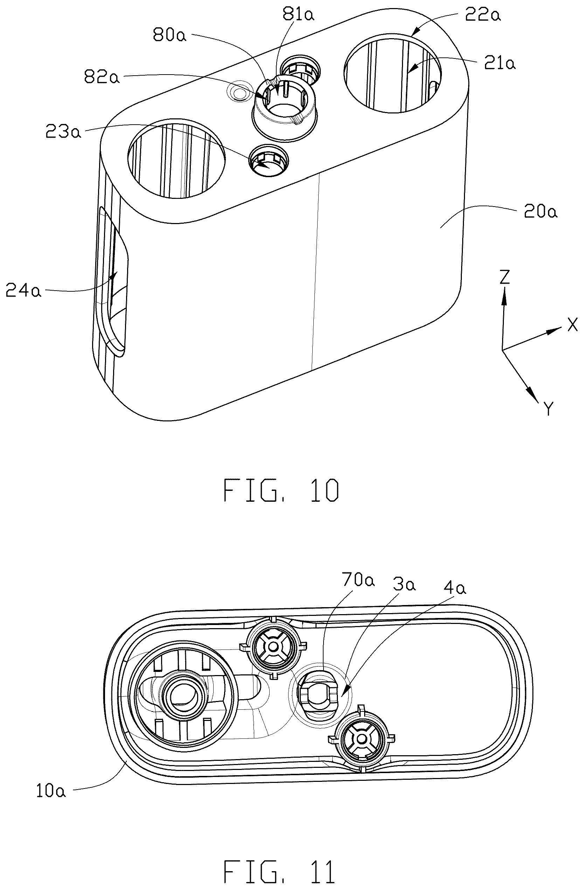

In conjunction with FIGS. 10 to 11 again, and also referring to FIGS. 1 and 2, in some embodiments, an inner circumferential surface of the rotating groove 81a defines a lubrication groove 82a, which is configured to store lubricating medium. The lubricating medium may be grease or oil paste, etc.

The number of lubrication grooves 82a is multiple, and the multiple lubrication grooves 82a are evenly spaced around the rotation axis H. The lubrication grooves 82a extend from the wall of the first groove body 811a to an outside of the rotating base 80a along a radial direction of the rotating base 80a. Additionally, an extension direction of each of the lubrication grooves 82a is parallel to the first direction Z, and an end of each of the lubrication grooves 82a close to the mouthpiece 10a in the first direction Z extends to and penetrates an end surface of the rotating base 80a close to the mouthpiece 10a, so as to facilitate the injection of lubricating medium into the lubrication grooves 82a. The lubricating medium is located between the rotating component 70a and a wall of the rotating groove 81a, which not only reduces the wear of the rotating component 70a and increases its service life, but also improves the smoothness of the rotating component 70a when rotating in the rotating groove 81a.

A blocking part 3a protrudes from the side of the mouthpiece 10a close to the connecting component 20a. Along the first direction Z, the blocking part 3a protrudes from a bottom wall of the empty groove 13a towards the connecting component 20a, and the blocking part 3a is approximately a circular ring structure. The blocking part 3a is arranged around an outer circumferential surface of the rotating component 70a, and the blocking part 3a is spaced apart from the outer circumferential surface of the rotating component 70a to form an activity cavity 4a between the blocking part 3a and the rotating component 70a. The rotating base 80a is movably arranged in the activity cavity 4a. The activity cavity 4a can store the lubricating medium overflowing from the rotating groove 81a. Thus, when the rotating component 70a rotates in the rotating groove 81a, part of the lubricating medium is squeezed and overflows from the rotating groove 81a, and the overflowing lubricating medium enters the activity cavity 4a for temporary storage, thereby preventing the lubricating medium from entering the empty groove 13a by the blocking part 3a.

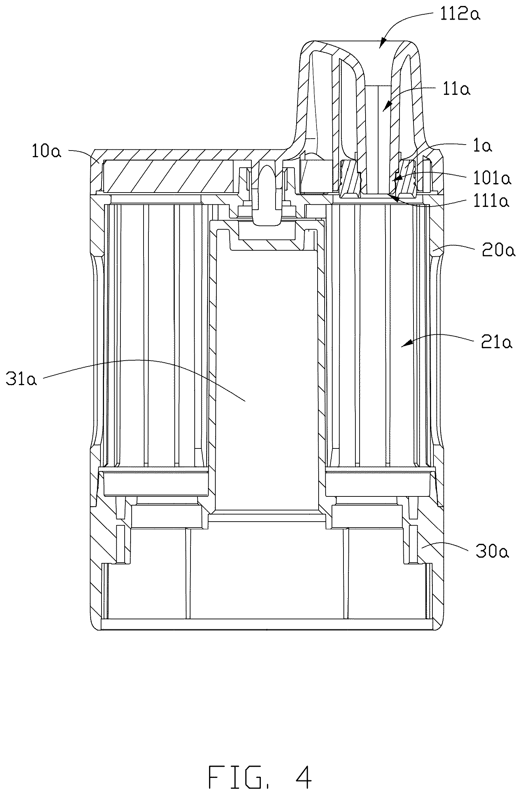

In conjunction with FIG. 8 again, and also referring to FIGS. 2 and 4, in some embodiments, the shell structure 100a may further include an oil-absorbing member 1a, which is connected to the mouthpiece 10a or the connecting component 20a. The oil-absorbing member 1a is configured to absorb the lubricating medium overflowing from the rotating groove 81a.

In the illustrated embodiment, the number of oil-absorbing members 1a is set to two, and the two oil-absorbing members 1a are over-tightened in the empty groove 13a. Along the second direction X, the two oil-absorbing members 1a are spaced apart to form an installation space 2a between them, and the installation space 2a is used for installing the rotating component 70a and the blocking part 3a. If part of the lubricating medium overflows from the activity cavity 4a, the lubricating medium will be adsorbed by the oil-absorbing member 1a, thereby preventing the lubricating medium from overflowing from the mouthpiece 10a and affecting the user's use.

It is worth noting that some aerosols will encounter cold air outside the air flow channel 21a and condense to form condensate liquid after leaving the air flow channel 21a. The condensate liquid will accumulate on the end surface of the connecting component 20a close to the mouthpiece 10a. The oil-absorbing member 1a arranged in the empty groove 13a can also adsorb the condensate liquid formed by the condensation of aerosols.

Furthermore, by overfitting the oil-absorbing member 1a into the empty slot 13a, the oil-absorbing member 1a can rotate synchronously along with the mouthpiece 10a. During the rotation of the oil-absorbing member 1a along with the mouthpiece 10a, the oil-absorbing member 1a will push the condensate liquid on the end surface of the connecting component 20a close to the mouthpiece 10a to move on that end surface. Meanwhile, the oil-absorbing member 1a can absorb all the condensate liquid on the end surface of the connecting component 20a close to the mouthpiece 10a during one full rotation, thereby preventing the condensate liquid from overflowing to the outside of the mouthpiece 10a and the connecting component 20a.

In some embodiments, along the first direction Z, the oil-absorbing member 1a and the end surface of the connecting component 20a close to the mouthpiece 10a may be spaced apart from each other, and a distance between them does not exceed 0.2mm, which ensures that the oil-absorbing member 1a can clean the condensate liquid on the end surface of the connecting component 20a close to the mouthpiece 10a without coming into contact with the connecting component 20a and generating frictional resistance that hinders the rotation of the mouthpiece 10a.

Additionally, a through hole 101a may be formed in the oil-absorbing member 1a, which extends through the oil-absorbing member 1a along the first direction Z. The through hole 101a allows a convex column including the inhalation channel 11a of the suction part 12a to pass through, thus connecting the airflow channel 21a and the inhalation channel 11a and ensuring that the aerosol can pass through the oil-absorbing member 1a smoothly.

It can be understood that in other embodiments, the number of oil-absorbing members 1a can also be set to one, and the only one oil-absorbing member 1a is overfitted into the empty slot 13a. Additionally, an installation space 2a is formed in the oil-absorbing member 1a and extends through the oil-absorbing member 1a along the first direction Z.

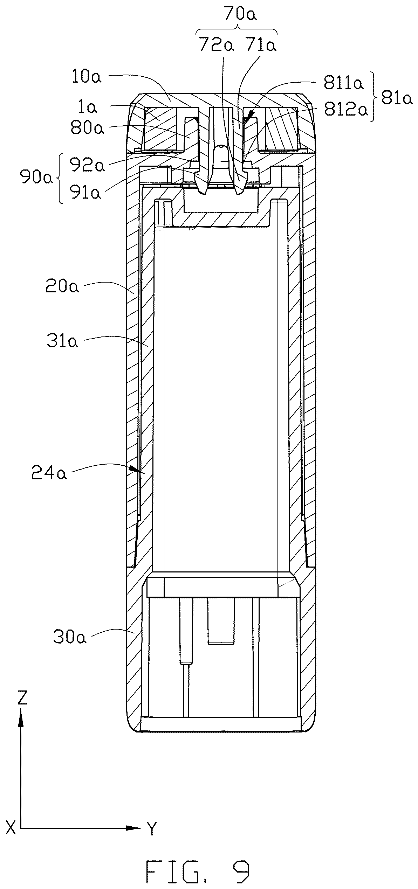

In conjunction with FIGS. 10 to 13 again, and also referring to FIGS. 8 and 9, in some embodiments, the connecting component 20a is approximately columnar in shape, and two first accommodation slots 23a are formed on the end surface of the connecting component 20a close to the mouthpiece 10a. Along the first direction Z, each of the first accommodation slots 23a extends from the end surface of the connecting component 20a close to the mouthpiece 10a towards the support component 30a. The two first accommodation slots 23a are located on opposite sides of the rotating base 80a, respectively.

The two first magnetic components 40a are respectively accommodated in the two first accommodation slots 23a and are clamped into the first accommodation slots 23a to achieve the connection between the first magnetic components 40a and the connecting component 20a.

It can be understood that in other embodiments, the first magnetic components 40a may also be connected to the connecting component 20a through other methods such as bonding.

An installation part 14a protrudes from an end of the mouthpiece 10a close to the connecting component 20a and extends into the first accommodation slot 23a or is located outside the connecting component 20a. The first magnetic component 40a is arranged on an end surface of the installation part 14a close to the connecting component 20a.

Along the first direction Z, the installation part 14a protrudes from the bottom wall of the empty slot 13a towards the connecting component 20a. The end surface of the installation part 14a close to the connecting component 20a is provided with a second accommodation slot 140a, and the second magnetic component 50a can be clamped into the second accommodation slot 140a, thereby achieving the connection between the second magnetic component 50a and the installation part 14a. The number of installation parts 14a may be two, and the two installation parts 14a are located on opposite sides of the rotating part 70a, respectively. The two second magnetic components 50a are accommodated in the second accommodation slots 140a of the two installation parts 14a, respectively.

It can be understood that in other embodiments, the second magnetic components 50a may also be connected to the installation parts 14a through other methods such as bonding.

In the illustrated embodiment, an end of the installation part 14a close to the connecting component 20a is spaced apart from the connecting component 20a, and a distance between them is no more than 0.3mm, so that a distance between the first magnetic component 40a and the second magnetic component 50a is no more than 0.3mm.

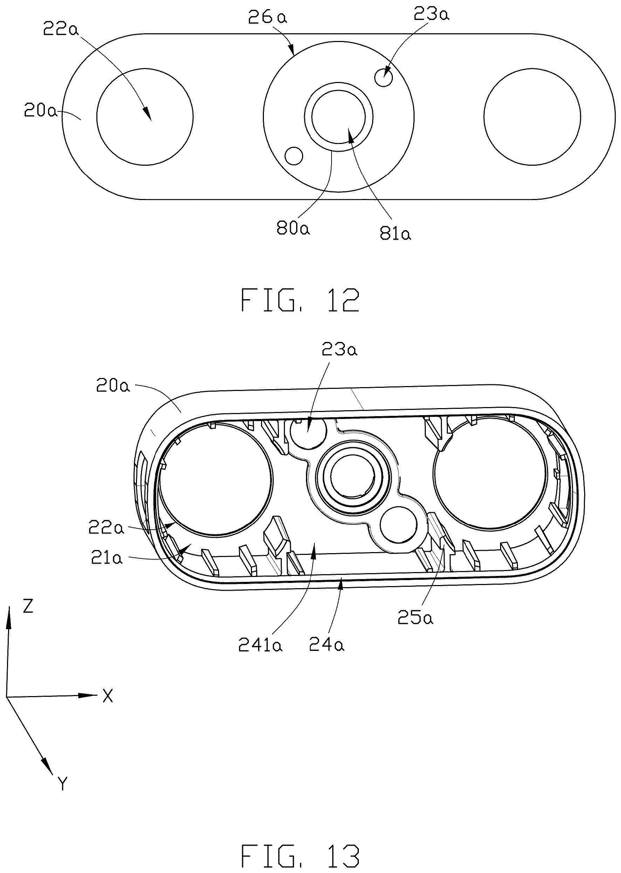

It can be understood that in other embodiments, the installation part 14a may partially extend into the connecting component 20a. In some embodiments, as shown in FIG. 12, the end surface of the connecting component 20a close to the mouthpiece 10a is provided with a third accommodation groove 26a. The rotating base 80a is located in the third accommodation groove 26a, and both of the two first accommodation grooves 23a extend from a bottom wall of the third accommodation groove 26a. When the mouthpiece 10a is connected to the connecting component 20a, the rotating part 70a and the installation part 14a are both located in the third accommodation groove 26a, which can prevent the installation part 14a from interfering with the rotation of the mouthpiece 10a.

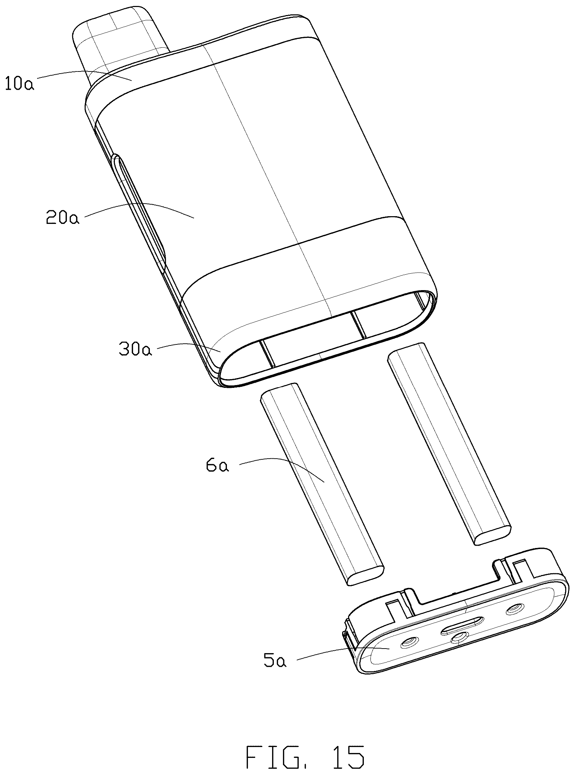

In conjunction with FIGS. 13 and 15 again, and also referring to FIGS. 2 and 4, in some embodiments, an end surface of the connecting component 20a close to the support component 30a is provided with a fourth accommodation groove 24a. Along the first direction Z, the fourth accommodation groove 24a extends from the end surface of the connecting component 20a close to the support component 30a towards the mouthpiece 10a, and extends to communicate with the first accommodation groove 23a.

An extension part 31a protrudes from an end surface of the support component 30a close to the connecting component 20a, and an extension direction of the extension part 31a is parallel to the first direction Z. The extension part 31a is located in the fourth accommodation groove 24a, and an end surface of the extension part 31a away from the support component 30a abuts a bottom wall of the fourth accommodation groove 24a, so as to support the connecting component 20a through the extension part 31a. The end of the support component 30a away from the extension part 31a is at least partially located outside the fourth accommodation groove 24a, so that a part of the support component 30a can abut an end of the connecting component 20a away from the mouthpiece 10a, and an end of the fourth accommodation groove 24a away from the mouthpiece 10a is closed by the support component 30a.

In the illustrated embodiment, the aerosol generation assembly 6a is located in the fourth accommodation groove 24a, and the aerosol generation assembly 6a is clamped between the bottom wall of the fourth accommodation groove 24a and the support component 30a.

In the illustrated embodiment, the end surface of the extension part 31a away from the support component 30a is provided with two fifth accommodation grooves 310a. Along the first direction Z, each of the fifth accommodation grooves 310a extends from the end surface of the extension part 31a away from the support component 30a towards the support component 30a. The two fifth accommodation grooves 310a are respectively connected to the two first accommodation grooves 23a, and the two third magnetic components 60a are respectively installed in the two fifth accommodation grooves 310a. The third magnetic components 60a may be connected to the extension part 31a by snap fitting or adhesive bonding.

In conjunction with FIGS. 13 and 15 again, and also referring to FIGS. 2 and 4, in some embodiments, a partition part 25a protrudes from an inner circumferential surface of the fourth accommodation groove 24a. The partition part 25a is approximately a long strip structure, and an extension direction of the partition part 25a is parallel to the first direction Z.

The number of the partition parts 25a is four, and the four partition parts 25a are approximately distributed in a rectangular shape. Two partition parts 25a of the four partition parts 25a are connected to an inner wall of the fourth accommodation groove 24a along the third direction Y, and the other two partition parts 25a of the four partition parts 25a are connected to another inner wall of the fourth accommodation groove 24a along the third direction Y. In addition, the two partition parts 25a located on the same inner wall of the fourth accommodation groove 24a are spaced apart in the second direction X, so that a receiving space 241a is formed between the four partition parts 25a. The extension part 31a is located in the receiving space 241a, and an outer circumferential surface of the extension part 31a abuts the four partition parts 25a, which ensure that the extension part 31a does not shake in the receiving space 241a.

Furthermore, an arrangement of the four partition parts 25a divides a space of the fourth accommodating groove 24a into two airflow channels 21a. Along the second direction X, the two airflow channels 21a are located on opposite sides of the four partition parts 25a, respectively. One of the two aerosol generation assemblies 6a is installed in one of the two airflow channels 21a, and the other aerosol generation assembly 6a is installed in the other airflow channel 21a.

In some embodiments, two airflow ports 22a may be formed on the end surface of the connecting component 20a close to the mouthpiece 10a. One of the two airflow ports 22a is in communication with one of the two airflow channels 21a, and the other airflow port 22a is in communication with the other airflow channel 21a. Along the first direction Z, each of the airflow ports 22a extends from the end surface of the connecting component 20a close to the mouthpiece 10a towards a side of the connecting component 20a away from the mouthpiece 10a to communicate with the corresponding airflow channels 21a.

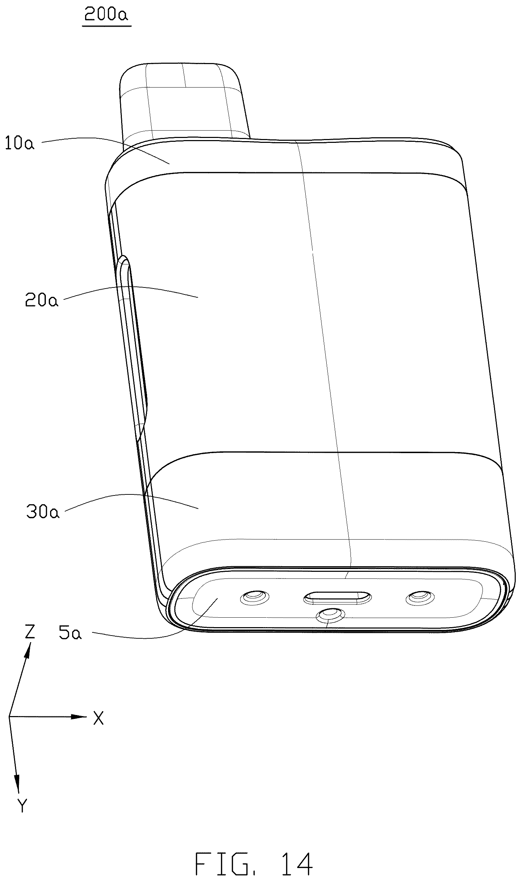

Referring to FIGS. 14 and 15, and in combination with FIG. 1, the present application provides an aerosol device 200a in Embodiment 1, which includes the above-mentioned shell structure 100a and aerosol generation assemblies 6a. The aerosol generation assemblies 6a are removably accommodated in the connection component 20a of the above-mentioned shell structure 100a.

In addition, the aerosol device 200a may also include a power supply assembly 5a, which is installed in the support component 30a and is electrically connected to the two aerosol generation assemblies 6a to supply power to the two aerosol generation assemblies 6a through the power supply component 5a. The power supply component 5a includes a power supply element and a conductive element. The power supply element is a battery or other component capable of generating electrical energy. One end of the conductive element is electrically connected to the power supply element, and the other end is electrically connected to the two aerosol generation assemblies 6a to supply power to the two aerosol generation assemblies 6a through the power supply element.

It can be understood that in other embodiments, the power supply component 5a can also be installed at an end of the support component 30a away from the connecting component 20a.

### Embodiment 2

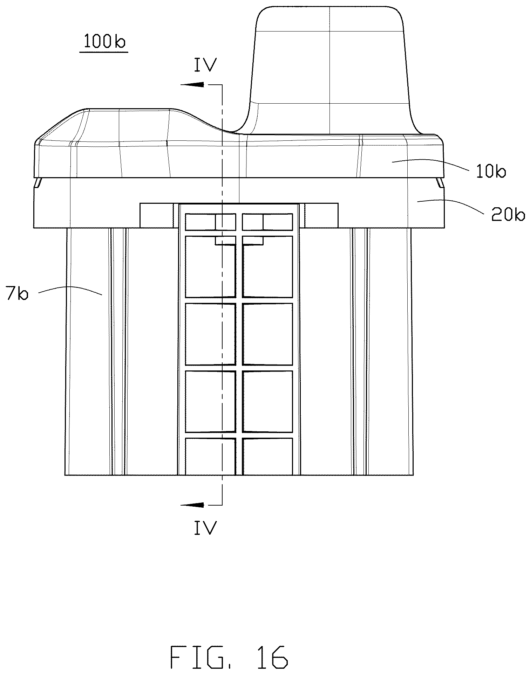

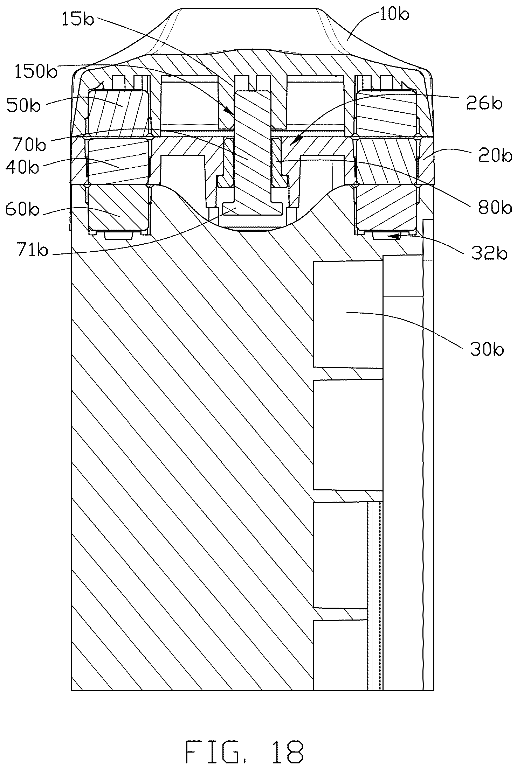

Referring to FIGS. 16 to 18, Embodiment 2 provides a shell structure 100b, which is applied to an aerosol device 200b. The aerosol device 200b includes the shell structure 100b and aerosol generation assemblies 6b. The shell structure 100b includes a mouthpiece 10b, a connecting component 20b, a support component 30b, first magnetic components 40b, second magnetic components 50b, third magnetic components 60b, a rotating component 70b, and a rotating base 80b.

Along the first direction Z, the support component 30b, the connecting component 20b, and the mouthpiece 10b are arranged in sequence, and the mouthpiece 10b can rotate relative to the connecting component 20b. Along the first direction Z, an empty slot 13b is formed on an end of the mouthpiece 10b close to the connecting component 20b. A connection protrusion 15b protrudes from a bottom wall of the empty slot 13b, and extends from the bottom wall of the empty slot 13b towards the connecting component 20b along the first direction Z. An end of the connection protrusion 15b close to the connecting component 20b has a first connection recess 150b, and the rotating component 70b is partially accommodated in the first connection recess 150b and is removably connected to the connection protrusion 15b.

A second connection recess 26b is formed on an end of the connecting component 20b close to the mouthpiece 10b, and the second connection recess 26b extends along the first direction Z to penetrate the connecting component 20b. The rotating base 80b is inserted into the second connection recess 26b and is removably connected to the connecting component 20b.

In some embodiments, the rotating component 70b may be approximately a cylindrical structure, and the rotating base 80b may be approximately a hollow cylindrical structure. The rotating component 70b is inserted through the rotating base 80b and can rotate relative to the rotating base 80b. An end of the rotating component 70b away from the mouthpiece 10b is located outside the rotating base 80b, and a limiting protrusion 71b is arranged on an outer circumferential surface of the end of the rotating component 70b away from the mouthpiece 10b. An outer diameter of the limiting protrusion 71b is greater than an inner diameter of the rotating base 80b, so that the rotating component 70b is limited in the first direction Z by the rotating base 80b pressing against the limiting protrusion 71b, thereby preventing the rotating component 70b from moving away from the connection component 20b and detaching from the rotating base 80b.

In the illustrated embodiment, along the first direction Z, two air flow ports 22b are arranged on an end surface of the connecting component 20b close to the mouthpiece 10b, and two air flow channels 21b are arranged on the end of the connecting component 20b close to the support component 30b. The two air flow channels 21b are connected to the two air flow ports 22b, respectively.

The support component 30b abuts an end of the connecting component 20b away from the mouthpiece 10b, thereby supporting the connecting component 20b by the support component 30b. An end surface of the support component 30b close to the connecting component 20b is provided with a fifth accommodating groove 32b, and the third magnetic components 60b are accommodated in the fifth accommodating groove 32b.

Along the second direction X, opposite sides of the support component 30b has accommodating grooves 31b. The two accommodating grooves 31b are respectively connected to the two airflow channels 21b, and the two aerosol generation assemblies 6b are respectively accommodated in the two accommodating grooves 31b.

In some embodiments, the shell structure 100b may further include an outer shell 7b, which covers an outer side of the support component 30b to seal the two accommodating grooves 31b, thereby ensuring the safety of the two aerosol generation assemblies 6b located in the two accommodating grooves 31b.

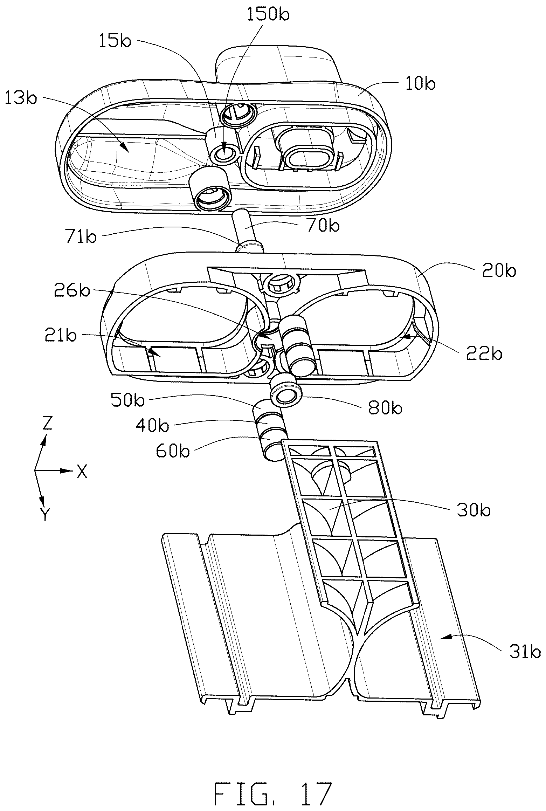

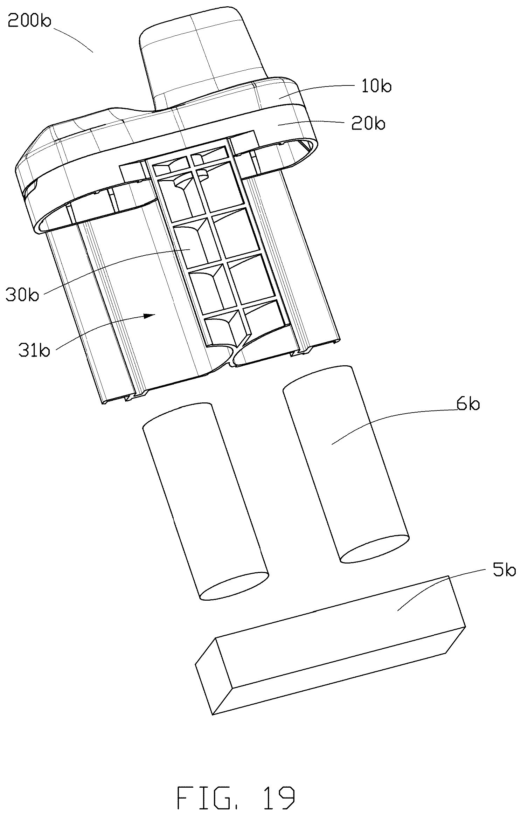

Referring to FIG. 19 and also referring to FIGS. 16 and 17, the Embodiment 2 of the present application also provides an aerosol device 200b, which includes the above-mentioned shell structure 100b and the aerosol generation assemblies 6b. The aerosol generation assemblies 6b are removably accommodated in the support component 30b of the shell structure 100b.

In addition, the aerosol device 200b may further includes a power assembly 5b, which is installed on a side of the support component 30b away from the connecting component 20b and located inside the outer shell 7b. The power assembly 5b is electrically connected to the two aerosol generation assemblies 6b to supply power to the two aerosol generation assemblies 6b. The power assembly 5b includes a power supply component and a conductive component. The power supply component may b a battery or other component that can generate electrical energy. One end of the conductive component is electrically connected to the power supply component, and the other end is electrically connected to the two aerosol generation assemblies 6b to supply power to the two aerosol generation assemblies 6b through the power supply component.

Except for the above structure, the other structures of the aerosol device 200b provided in the Embodiment 2 are the same as those in the Embodiment 1 and will not be described in detail.

In the above text, the specific embodiments of the present application have been described with reference to the drawings. However, it should be understood by those skilled in the art that various changes and modifications can be made to the specific embodiments without departing from the scope of the present application. These changes and modifications all fall within the scope defined by the present application.

## Claims

1. A shell structure (100a, 100b), applied in an aerosol device (200a, 200b), the shell structure (100a, 100b) comprising:
a mouthpiece (10a, 10b) provided with an inhalation channel (11a);
a connecting component (20a, 20b) arranged on a side of the mouthpiece (10a, 10b), wherein the connecting component (20a, 20b) is provided with an airflow channel (21a, 21b), and the airflow channel (21a, 21b) is connected to an air inlet end (111a) of the inhalation channel (11a);
a support component (30a, 30b) configured to support the connecting component (20a, 20b);
a first magnetic component (40a, 40b) arranged on the connecting component (20a, 20b);
a second magnetic component (50a, 50b) arranged on the mouthpiece (10a, 10b); and
a third magnetic component (60a, 60b) arranged on the support component (30a, 30b);
wherein at least one of the connecting component (20a, 20b) and the support component (30a, 30b) is configured to accommodate an aerosol generation assembly (6a, 6b) of the aerosol device (200a, 200b), the airflow channel (21a, 21b) is configured to receive an aerosol generated by the aerosol generation assembly (6a, 6b), the mouthpiece (10a, 10b) is magnetically connected to the connecting component (20a, 20b) through a magnetic force between the third magnetic component (60a, 60b) and the first magnetic component (40a, 40b).

2. The shell structure (100a, 100b) of claim 1, wherein the shell structure (100a, 100b) comprises at least two first magnetic components (40a, 40b) comprising the first magnetic component (40a, 40b), at least two second magnetic components (50a, 50b) comprising the second magnetic component (50a, 50b), and at least two third magnetic components (60a, 60b) comprising the third magnetic component (60a, 60b);
the at least two second magnetic components (50a, 50b) correspond one-to-one to the at least two first magnetic components (40a, 40b), and the at least two third magnetic components (60a, 60b) correspond one-to-one to the at least two first magnetic components (40a, 40b).

3. The shell structure (100a, 100b) of claim 1, wherein a first accommodation slot (23a) is arranged on an end surface of the connecting component (20a, 20b) close to the mouthpiece (10a, 10b), the first magnetic component (40a, 40b) is located in the first accommodation slot (23a);
an installation part (14a) protrudes from an end of the mouthpiece (10a, 10b) close to the connecting component (20a, 20b) and extends into the first accommodation slot (23a) or is located outside the connecting component (20a, 20b), and the first magnetic component (40a, 40b) is arranged an end surface of the installation part (14a) close to the connecting component (20a, 20b).

4. The shell structure (100a, 100b) of claim 1, wherein the connecting component (20a, 20b) is provided with at least two airflow channels (21a, 21b), the mouthpiece (10a, 10b) is configured to be rotatable relative to the connecting component (20a, 20b), thereby allowing the inhalation channel (11a) of the mouthpiece (10a, 10b) to be selectively connected to any one of the at least two airflow channels (21a, 21b).

5. The shell structure (100a, 100b) of claim 4, wherein the shell structure (100a, 100b) defines a rotation axis (H), the mouthpiece (10a, 10b) is rotatable around the rotation axis (H), the number of the at least two airflow channels (21a, 21b) is 2N, N is a positive integer, the shell structure (100a, 100b) comprises N groups of first magnetic components (40a, 40b) comprising the first magnetic component (40a, 40b), each group of the N groups of first magnetic components (40a, 40b) comprises at least two first magnetic components (40a, 40b), the at least two first magnetic components (40a, 40b) comprises two first magnetic components (40a, 40b) symmetrically arranged with respect to the rotation axis (H).

6. The shell structure (100a, 100b) of claim 4, wherein the shell structure (100a, 100b) defines a rotation axis (H), the mouthpiece (10a, 10b) is rotatable around the rotation axis (H), the number of the at least two airflow channels (21a, 21b) is 2N+1, N is a positive integer, the shell structure (100a, 100b) comprises N+1 groups of first magnetic components (40a, 40b) comprising the first magnetic component (40a, 40b), each group of the N+1 groups of first magnetic components (40a, 40b) comprises at least one first magnetic component (40a, 40b), the N+1 groups of first magnetic components (40a, 40b) are evenly spaced around the rotation axis (H).

7. The shell structure (100a, 100b) of claim 4, wherein the shell structure (100a, 100b) further comprises a rotating component (70a, 70b) and a rotating base (80a, 80b), the rotating component (70a, 70b) is connected to a side of the mouthpiece (10a, 10b) close to the connecting component (20a, 20b), the rotating base (80a, 80b) is connected to the connecting component (20a, 20b);
the rotating base (80a, 80b) comprises a rotating groove (81a), and at least part of the rotating component (70a, 70b) is rotatably arranged in the rotating groove (81a).

8. The shell structure (100a, 100b) of claim 7, wherein the shell structure (100a, 100b) further comprises a limiting structure (90a), the rotating component (70a, 70b) rotatably cooperates with the rotating groove (81a), the limiting structure (90a) is configured to restrict a movement of the rotating component (70a, 70b) along an axis of the rotating component (70a, 70b).

9. The shell structure (100a, 100b) of claim 8, wherein the limiting structure (90a) further comprises a first limiting part (91a) and a second limiting part (92a), the first limiting part (91a) is arranged on the rotating component (70a, 70b), the second limiting part (92a) is arranged on the rotating base (80a, 80b), the first limiting part (91a) and the second limiting part (92a) abut against each other along the axis of the rotating component (70a, 70b) when the rotating component (70a, 70b) rotatably cooperates with the rotating groove (81a).

10. The shell structure (100a, 100b) of claim 7, wherein an inner circumferential surface of the rotating groove (81a) defines a lubrication groove (82a), and the lubrication groove (82a) is configured to store lubricating medium.

11. The shell structure (100a, 100b) of claim 10, wherein a blocking part (3a) protrudes from the side of the mouthpiece (10a, 10b) close to the connecting component (20a, 20b), the blocking part (3a) is arranged around an outer circumferential surface of the rotating component (70a, 70b), the blocking part (3a) is spaced apart from the outer circumferential surface of the rotating component (70a, 70b) to form an activity cavity (4a) between the blocking part (3a) and the rotating component (70a, 70b), the rotating base (80a, 80b) is movably arranged in the activity cavity (4a);
the activity cavity (4a) is configured to store the lubricating medium overflowing from the rotating groove (81a).

12. The shell structure (100a, 100b) of claim 10, wherein the shell structure (100a, 100b) further comprises an oil-absorbing member (1a), the oil-absorbing member (1a) is connected to the mouthpiece (10a, 10b) or the connecting component (20a, 20b), the oil-absorbing member (1a) is configured to absorb the lubricating medium overflowing from the rotating groove (81a), and/or the oil-absorbing member (1a) is configured to absorb condensate liquid formed after the aerosol condenses.

13. The shell structure (100a, 100b) of claim 1, wherein the shell structure (100a, 100b) further comprises an oil-absorbing member (1a), the oil-absorbing member (1a) is connected to the side of the mouthpiece (10a, 10b) close to the connecting component (20a, 20b), the oil-absorbing member (1a) is configured to absorb condensate liquid formed after the aerosol condenses, a through hole (101a) is formed in the oil-absorbing member (1a) and connects the airflow channel (21a, 21b) and the inhalation channel (11a).

14. The shell structure (100a, 100b) of claim 13, wherein the mouthpiece (10a, 10b) is configured to be rotatable relative to the connecting component (20a, 20b), the oil-absorbing member (1a) is rotatable relative to the connecting component (20a, 20b) during a rotation of the mouthpiece (10a, 10b);
the oil-absorbing member (1a) is configured to absorb the condensate liquid on an end surface of the connecting component (20a, 20b) close to the mouthpiece (10a, 10b) during a rotation of the oil-absorbing member (1a) relative to the connecting component (20a, 20b).

15. An aerosol device (200a, 200b) comprising:
the shell structure (100a, 100b) of any one of claims 1 to 14; and
an aerosol generation assembly (100a, 100b), wherein the aerosol generation assembly (100a, 100b) are removably accommodated in the connection component (20a, 20b) of the shell structure (100a, 100b) or the support component (30a, 30b) of the shell structure (100a, 100b).
